# EUROPEAN PATENT APPLICATION

(11) **EP 4 464 775 A1**
(43) Date of publication of application: **20.11.2024**
(21) Application number: 23740494.2
(22) Date of filing: 13.01.2023
(51) Int. Cl.: C12N 5/00, C12N 5/077

(54) **PHENOL GROUP-MODIFIED DECELLULARIZED MUSCLE TISSUE-DERIVED EXTRACELLULAR MATRIX FOR MUSCLE REGENERATION, AND METHOD FOR PREPARING SAME**

(30) Priority: 13.01.2022 KR 20220005526
(71) Applicant: Industry-Academic Cooperation Foundation, Yonsei University, Seoul 03722 (KR)
(72) Inventor: CHO, Seung Woo, Seoul 03722 (KR); CHOI, Soo Jeong, Seoul 04731 (KR); LEE, Mi Jeong, Seoul 03938 (KR); CHOI, Yi Sun, Seoul 03722 (KR); AN, Soo Hwan, Seoul 03722 (KR)
(74) Representative: Mewburn Ellis LLP
(86) International application number: PCT/KR2023/000638
(87) International publication number: WO 2023/136651

(57) **Abstract**

The present invention relates to a phenol-modified decellularized muscle tissue-derived extracellular matrix (MEM) for muscle regeneration and a method for preparing same. The phenol group-modified decellularized MEM of the present invention is characterized by containing components similar to original proteins of muscle tissue for muscle regeneration and forming a hydrogel having a stable porous nanofiber structure through dimerization crosslinking between phenol groups, wherein the hydrogel can effectively treat muscle injury or muscle diseases by being administered or implanted into muscle tissue without causing side effects such as an immune response or liver or kidney dysfunction.

## Description

### TECHNICAL FIELD

The present invention relates to a phenol group-modified decellularized muscle tissue-derived extracellular matrix (MEM) for muscle regeneration, a method for preparing the same, and a use of the same.

### BACKGROUND ART

Muscle, which is an organ that enables movement by being attached to the bones that make up the skeleton of the body, is responsible for the movement and manipulation of the body, maintaining posture and extending joints, and plays an important role in maintaining life by moving the heart and internal organs. Muscles may be broadly classified into skeletal muscles, cardiac muscles, and visceral muscles, and according to the functions, they may also be classified into voluntary muscles, of which contraction may be controlled by a person's own will, and involuntary muscles, of which contraction may not be controlled, and according to the shapes, they may also be classified into striated muscles and smooth muscles.

Like other organs, these muscles may develop various congenital or acquired diseases, and damage by trauma may also occur. The types of muscle diseases are very diverse, but examples include muscular dystrophy, inflammatory muscle lesions (myositis), myotonia, metabolic muscle lesions, periodic paralysis, and congenital muscle lesions.

As prior arts related to the diagnosis, prevention, and treatment of muscle diseases or muscle damage, Korean Patent Publication No. 10-2019-0108417 discloses a dual functional peptide having a cell permeability and a muscle regeneration ability and a use thereof, and Korean Patent Publication No. 10-2018-0138171 discloses a method of screening therapeutic agent for muscular dystrophy or sarcopenia. In addition to the above-mentioned prior arts, various muscle disease treatment technologies are being studied and utilized, but in reality, there is an increasing demand for a treatment technology using a composition having high similarity to muscle cells and tissues.

Under this background, the present inventors made diligent efforts to solve the above problems, and as a result, they confirmed that a more tissue-friendly muscle regeneration effect can be derived by utilizing a phenol group-modified decellularized MEM and completed the present invention.

### DETAILED DESCRIPTION OF THE INVENTION

### TECHNICAL PROBLEM

An object of the present invention is to provide a hydrogel composition including a decellularized muscle tissue-derived extracellular matrix (MEM) modified with one or more selected from the group consisting of a catechol group and a pyrogallol group.

In addition, another object of the present invention is to provide a pharmaceutical composition for preventing or treating muscle disease or muscle damage, including the hydrogel composition.

In addition, still another object of the present invention is to provide a method for preventing or treating muscle diseases, including administering or implanting the hydrogel composition to a subject other than a human in need of administration or implantation thereof.

### TECHNICAL SOLUTION

One aspect of the present invention relates to a hydrogel composition including a decellularized MEM modified with one or more selected from the group consisting of a catechol group and a pyrogallol group.

Another object of the present invention relates to a pharmaceutical composition for preventing or treating muscle disease or muscle damage, including the hydrogel composition.

Still another object of the present invention relates to a method for preventing or treating muscle diseases, including administering or implanting the hydrogel composition to a subject other than a human in need of administration or implantation thereof.

### ADVANTAGEOUS EFFECTS

The phenol group-modified decellularized MEM of the present invention is characterized in that it contains components similar to original proteins of muscle tissue for muscle regeneration and is able to form a hydrogel having a stable porous nanofiber structure through dimerization crosslinking between phenol groups, wherein the hydrogel can effectively treat muscle injury or muscle disease by being administered or implanted into muscle tissue without causing side effects such as an immune response or liver or kidney dysfunction.

### DESCRIPTION OF THE DRAWINGS

FIG. 1 shows a process of synthesizing catechol group (CA)/pyrogallol group (PG)-modified muscle tissue-derived extracellular matrix (MEM-CA, MEM-PG) derivatives by modifying muscle tissue-derived extracellular matrix (MEM) that has undergone decellularization and solubilization with a catechol group and a pyrogallol group through a chemical reaction using 1-ethyl-3-(3-dimethylaminopropyl)carbodiimide (EDC)/N-hydroxysuccinimide (NHS).
FIG. 2 shows the results of producing decellularized muscle tissue (MEM) using porcine skeletal muscle tissue and confirming the amount of double-stranded DNA (dsDNA), glycosaminoglycan (GAG), and collagen in the tissue to examine complete decellularization and good preservation of the extracellular matrix components.
FIG. 2a shows the results confirming the removal of nuclei, preservation of collagen components, and preservation of GAG components in MEM through a histological analysis.
FIGS. 2b, 2c, and 2d show the results of analyzing the remaining amounts of dsDNA, GAG, and collagen, respectively.
FIG. 3 shows the results of analyzing the extracellular matrix proteins in MEM.
FIG. 3a shows a schematic diagram of the component ratio of the matrisome protein of MEM.
FIG. 3b shows the results of schematically illustrating the 10 most abundantly detected matrisome proteins in the MEM.
FIG. 3c shows the results of conducting a gene ontology analysis on the matrisome proteins.
FIG. 3d shows the results of conducting a gene ontology analysis on proteins other than the matrisome.
FIG. 4 shows the results of comparing the analyzed proteome of the MEM-derived extracellular matrix and the proteome of human muscle to confirm whether MEM may provide a muscle microenvironment.
FIG. 4a shows the results of summarizing the major matrisome proteins remaining in the MEM even after a decellularization process.
FIG. 4b shows the results of comparing the number of muscle-specific proteins and matrisome proteins in the MEM and human muscle.
FIG. 5 shows the results of confirming the internal structure of MEM, MEM-CA, and MEM-PG hydrogels through scanning electron microscopy.
FIG. 6 shows the results of analyzing the chemical structures of MEM, MEM-CA, and MEM-PG solutions and MEM, MEM-CA, and MEM-PG hydrogels.
FIG. 7 shows the results of analyzing the degradation patterns of MEM, MEM-CA, and MEM-PG hydrogels under each concentration condition of 1% and 2%.
FIG. 8 shows the results of analyzing the mechanical properties of MEM, MEM-CA, and MEM-PG hydrogels under each concentration condition of 1% and 2% using rheological analysis equipment.
FIG. 8a shows the results of measuring the elastic modulus of MEM, MEM-CA, and MEM-PG hydrogels at each concentration.
FIG. 8b shows the results of comparing the results of measuring the average elastic modulus of MEM, MEM-CA, and MEM-PG hydrogels at each concentration.
FIG. 9 shows the results of an experiment for examining the injection force of hydrogel through injection of PBS, MEM-CA, and MEM-PG hydrogels at each concentration condition of 1% and 2%.
FIG. 10 shows the results of confirming the antioxidant function of MEM, MEM-CA, and MEM-PG hydrogels, and ascorbic acid.
FIG. 10a shows the results of confirming the antioxidant function of MEM, MEM-CA, and MEM-PG hydrogels, and ascorbic acid.
FIGS. 10b and 10C show the result of comparing the antioxidant effect measured in FIG. 10A.
FIG. 11 shows the results of evaluating the immunogenicity of each of MEM, MEM-CA, and MEM-PG hydrogels.
FIG. 11a shows the results of confirming the amount of secretion of the immune response factor tumor necrosis factor alpha(TNF-α) from macrophages when macrophages were co-cultured with MEM, MEM-CA, and MEM-PG hydrogels.
FIG. 11b shows the results of confirming the amount of secretion of the immune response factor interleukin 6(IL-6) from macrophages when macrophages were co-cultured with MEM, MEM-CA, and MEM-PG hydrogels.
FIG. 12 shows the results of confirming, through a histological analysis, whether an inflammatory reaction occurs after injecting MEM, MEM-CA, and MEM-PG hydrogels into mouse leg muscle tissue.
FIG. 13 shows the results of observing the expression of various immune cell markers after immunohistochemical staining to confirm biocompatibility after injecting the hydrogels into mice.
FIG. 14 shows the results of confirming the expression of each of inflammatory and anti-inflammatory macrophages after injecting the hydrogels into mice.
FIG. 15 shows the results of a complete blood count to determine whether an inflammatory reaction occurs when MEM, MEM-CA, and MEM-PG hydrogels are injected into mouse leg muscle tissue.
FIGS. 15a to 15h show graphs illustrating the results of measuring the white blood cells, lymphocytes, red blood cells, hemoglobin, hematocrit, mean corpuscular volume, mean corpuscular hemoglobin, mean corpuscular hemoglobin concentration, respectively.
FIG. 16 shows the results of conducting a serum analysis to determine whether systemic toxicity occurs when MEM, MEM-CA, MEM-PG hydrogel, or PBS is injected.
FIGS. 16a to 16g show the results of measuring the Mean platelet volume, blood urea nitrogen, creatinine, aspartate transaminase, alanine transaminase, total protein, and blood glucose, respectively.
FIG. 17 shows the results of observing tissue damage and immune response through a histological analysis of major organs collected after injecting MEM, MEM-CA, and MEM-PG hydrogels.
FIG. 18 shows the results illustrating the effect of increasing muscle mass when MEM, MEM-CA, and MEM-PG hydrogels and PBS were injected using a disuse skeletal muscle atrophy small animal model.
FIG. 18a shows the results of confirming the expression of various functional muscle markers through histology and immunohistochemical staining two weeks after injecting MEM, MEM-CA, MEM-PG hydrogels and PBS.
FIGS. 18b to 18F show graphs illustrating the results of measuring the indicators indicating an increase in muscle mass when MEM-CA and MEM-PG hydrogels and PBS were injected.
FIG. 19 shows the results of comparing the effect on functional muscle marker expression and muscle mass increase in the disuse skeletal muscle atrophy small animal model through histology and immunohistochemical staining after injecting the hydrogels (MEM-CA, MEM-PG) of the present invention, MEMs (HA-CA/MEM, HA-PG/MEM) using the hydrogels developed in previous studies, and hydrogels (Skin-CA, Skin-PG) derived from tissues other than muscle.
FIG. 20 shows the results of quantifying and comparing the effect of various hydrogels developed in previous studies and MEM, MEM-CA, and MEM-PG hydrogels on increase of muscle mass.
FIGS. 20a to 20g show graphs illustrating indicators that allow to confirm the increase in muscle mass after injecting various hydrogels.
FIG. 21 shows the results of analyzing and comparing proteomes of MEM and decellularized skin tissue-derived extracellular matrix (SkEM).
FIG. 21a shows the results of analyzing and comparing the component ratios of matrisome proteins between the proteomes of MEM and SkEM.
FIG. 21b shows the results of analyzing and comparing the types and amounts of proteins constituting MEM and SkEM.
FIG. 21c shows the results of analyzing and comparing the 10 most expressed matrisome proteins in SkEM.
FIG. 22 shows the results of analyzing and comparing proteomes of MEM and SkEM and comparing proteins related to muscle function.
FIG. 22a shows the results of conducting a principal component analysis for all proteins of each of MEM and SkEM.
FIG. 22b shows the results of comparing MEM and SkEM for proteins related to muscle tissue development through gene ontology.
FIG. 23 shows the results of a gene ontology analysis for proteins expressed only in MEM by performing a proteomic analysis of MEM and SkEM.
FIG. 24 shows the results about the muscle mass increasing effect that may be induced when injecting MEM, MEM-CA, and MEM-PG hydrogels in a sarcopenia animal model.
FIG. 25 shows the results of comparing factors related to muscle mass increase after injecting MEM, MEM-CA, and MEM-PG hydrogels.
FIG. 26 shows the results of confirming biocompatibility through a histological analysis and a blood analysis of major organs after injecting MEM, MEM-CA, MEM-PG hydrogels and PBS in a sarcopenia animal model.
FIG. 26a shows the results of confirming immune response and tissue necrosis through a histological analysis of major organs after injecting MEM, MEM-CA, and MEM-PG hydrogels in a sarcopenia animal model.
FIG. 26b shows the results of confirming systemic toxicity by analyzing kidney functions, liver functions, total protein, and blood glucose after injecting MEM, MEM-CA, and MEM-PG hydrogels in a sarcopenia animal model.

### BEST MODE

Hereinafter, the present invention will be described with reference to the attached drawings. However, the present invention can be implemented in various different forms, and thus is not limited to the embodiments described herein. When a part is described to "include" a certain component, unless specifically stated otherwise, this does not mean that other components are excluded, but that other components can be further provided.

Unless otherwise specified, the present invention may be implemented by conventional techniques commonly used in the fields of molecular biology, microbiology, protein purification, protein engineering, and DNA sequence and recombinant DNA within the capabilities of those skilled in the art. These techniques are known to those skilled in the art and are described in many standardized textbooks and reference works.

Unless otherwise defined in the present specification, all technical and scientific terms used have the same meaning as commonly understood by those skilled in the art.

Various scientific dictionaries including terms included in the present specification are well known and available in the art. Any methods and materials similar or equivalent to those described in the present specification are found to be useful in the practice or testing of the present invention, but some methods and materials are described. Since the present invention can be used in a variety of ways depending on the context in which it is used by those skilled in the art, the present invention is not limited to specific methodologies, protocols, and reagents. The invention is described in more detail below.

As one aspect of the present invention, a hydrogel composition including a decellularized muscle tissue-derived extracellular matrix (MEM) modified with one or more selected from the group consisting of a catechol group and a pyrogallol group is provided.

The "extracellular matrix" refers to a natural support for cell growth prepared through decellularization of tissues found in mammals and multicellular organisms. The extracellular matrix may be further processed through dialysis or crosslinking.

The extracellular matrix may be collagens, elastins, laminins, glycosaminoglycans, proteoglycans, chemoattractants, cytokines, and a mixture of structural and non-structural biomolecules that are not limited by a growth factor.

Since the decellularized muscle tissue contains actual muscle tissue-specific extracellular matrix components, it may provide a physical, mechanical, and biochemical environment for the tissue and is very efficient in promoting differentiation into muscle tissue cells and tissue-specific functionality.

The "hydrogel" is a material in which a liquid using water as a dispersion medium hardens through a sol-gel phase transition and loses fluidity and thus forms a porous structure, and it may be formed as a hydrophilic polymer with a three-dimensional network structure and a microcrystalline structure swells by containing water.

In the present invention, the hydrogel in the composition may be formed by cross-linking one or more selected from the group consisting of a catechol group and a pyrogallol group with which an extracellular matrix is modified.

In the present invention, when the extracellular matrix in the hydrogel composition is modified with a catechol group, a catechol group-modified decellularized MEM (MEM-CA derivative) may be contained in an amount of 0.1% to 4% or 1% to 2% by weight compared to the total volume of the hydrogel composition.

In the present invention, when the extracellular matrix in the hydrogel composition is modified with a pyrogallol group, a pyrogallol group-modified decellularized MEM (MEM-PG derivative) may be contained in an amount of 0.1% to 4% or 1% to 2% by weight compared to the total volume of the hydrogel composition.

In the present invention, the hydrogel composition may not further include a crosslinking agent.

In the present invention, the decellularized MEM may contain DNA at a concentration of 10 ng/mg or less, may contain GAG at a concentration of 6 to 8 µg/mg, and may contain collagen at a concentration of 28 to 30 µg/mg.

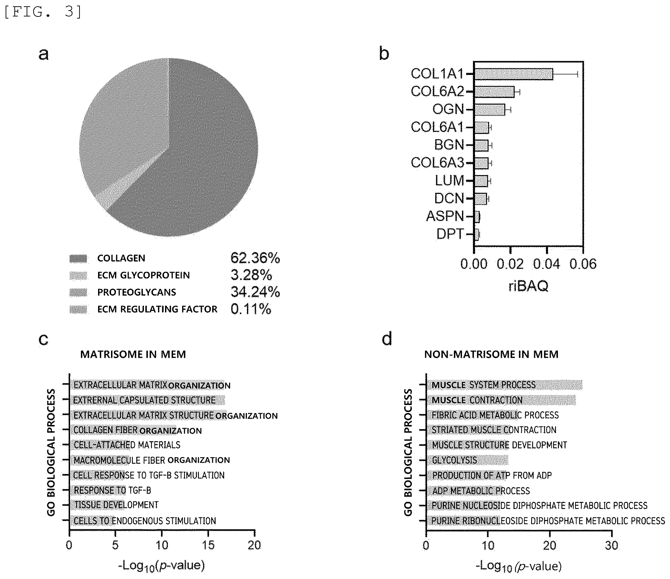

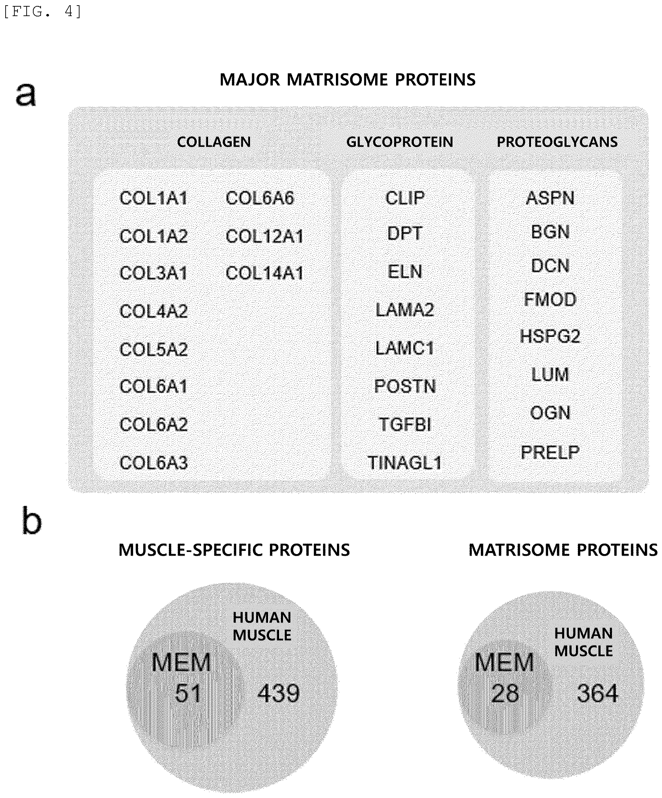

In the present invention, the decellularized MEM may contain 62% to 63% by weight of collagen, 3% to 4% by weight of ECM glycoprotein, and/or 34% to 35% by weight of proteoglycan. The collagen may mainly include COL1A1, COL6A2, COL6A1, and/or COL6A3. The decellularized MEM may include a material described in FIG. 3 or 4 as a component.

In the present invention, the hydrogel composition may be composed of a nanofibrous structure having a porous structure and may be cross-linked to each other by dimerization of catechol groups or pyrogallol groups with which the extracellular matrix is modified, thereby forming a stable hydrogels structure.

In the present invention, the hydrogel composition may have a G' (storage modulus) higher than G" (loss modulus). Specifically, when a decellularized MEM is modified with a catechol group and is contained in an amount of 0.1% to 4% by weight compared to the total volume of the hydrogel composition, the hydrogel composition may have an elastic modulus of 400 to 2000 Pa. In addition, when a decellularized MEM is modified with a pyrogallol group and is contained in an amount of 0.1% to 4% by weight compared to the total volume of the hydrogel composition, the hydrogel composition has an elastic modulus of 1200 to 2800 Pa. As the hydrogel composition contains an extracellular matrix modified with a catechol group or a pyrogallol group, it may have a higher elastic modulus compared to a hydrogel containing an unmodified extracellular matrix. In addition, when the hydrogel composition contains an extracellular matrix modified with a pyrogallol group, it may have a higher elastic modulus compared to a hydrogel containing an extracellular matrix modified with a catechol group.

In the present invention, the hydrogel composition may have a reactive oxygen species and radical scavenging ability and antioxidant ability and may have an improved reactive oxygen species and radical scavenging ability and antioxidant ability compared to a hydrogel composition containing an extracellular matrix that is not modified with a catechol group or a pyrogallol group.

In the present invention, the hydrogel composition may be characterized in that the hydrogel has a low possibility of inducing an immune response and causing tissue damage and has excellent biocompatibility.

As another aspect of the present invention, a composition for implanting into muscle tissue; and a pharmaceutical composition for preventing or treating muscle disease or muscle damage, including the hydrogel composition, are provided.

In the present invention, the muscle disease may be one or more muscle disease selected from muscular dystrophy, muscular atrophy, disuse skeletal muscle atrophy, sarcopenia, muscle loss, myalgia, myasthenia, muscle stiffness, ligament rupture, neurofibromatosis, tenosynovitis, polymyositis, dystonia, inflammatory muscle lesions (myositis), myotonia, metabolic muscle lesions, periodic paralysis, and congenital muscle lesions.

As another aspect of the present invention, the hydrogel composition may be a composition for forming an organoid, specifically, a composition for forming (culturing) a muscle tissue organoid.

As another aspect of the present invention, a composition for implanting into muscle tissue in a subject requiring implantation of muscle cells, including the hydrogel composition, is provided.

The "organoid" refers to a microscopic biological organ manufactured in the form of an artificial organ by culturing cells derived from tissues or pluripotent stem cells in a three-dimensional form.

The organoid is a three-dimensional tissue analogue including organ-specific cells that are generated from stem cells and self-organized (or self-patterned) in a manner similar to the *in vivo* state, and it may develop to a specific tissue by limited factor (e.g. growth factor) patterning

The organoid may have an anatomical structure having the original physiological characteristics of cells and mimicking the original state of a cell mixture (including not only limited cell types but also residual stem cells and adjacent physiological niches). Through a three-dimensional culture method, the organoid may obtain a functional organ-like morphology and tissue-specific functions with better arrangement of cells and cellular functions.

The composition of the present invention may be administered in a pharmaceutically effective amount.

In the present invention, "pharmaceutically effective amount" refers to an amount that is sufficient to treat a disease with a reasonable benefit/risk ratio applicable to medical treatment, and an effective dose level may be determined based on factors including the type and severity of a patient's disease, drug activity, sensitivity to drug, administration time, administration route and excretion rate, treatment duration, and drugs used simultaneously, and other factors well known in the medical field. The preferred dose of the composition of the present invention varies depending on the patient's conditions and weight, the degree of the disease, the drug form, the route and period of administration, but for a preferred effect, the pharmaceutical composition of the present invention may be administered at 0.0001 to 100 mg/kg per day. It may be administered once a day or in multiple times. The method of administration includes any conventional method in the art without limitation and may be administered, for example, orally, rectally, or by intravenous, intramuscular, or subcutaneous injection.

The pharmaceutical composition of the present invention may be prepared in a unit dose form or may be prepared by placing it in a multi-dose container by formulating it using a pharmaceutically acceptable carrier and/or excipient according to a method that may be easily performed by a person skilled in the art to which the present invention pertains.

As another aspect of the present invention, a method for increasing muscle weight, a method for preventing or treating muscle disease, or a method for implanting a hydrogel into muscle tissue, including administering or implanting the hydrogel composition to a subject in need of such administration or implantation.

In the present invention, the term "prevention and amelioration" refers to all actions that suppress or delay the onset of a muscle disease by the administration or implantation of the composition, and "treatment" refers to all actions that change for the better or beneficially change a muscle disease by the administration or implantation of the composition.

In the present invention, the hydrogel, extracellular matrix, and muscle disease are as described above.

In the present invention, the subject may mean an animal including or not including a human.

In the present invention, the method for preventing or treating muscle disease; or the method for implanting a hydrogel into muscle tissue may further include increasing the amount of expression of M2 macrophages more than the amount of expression of M1 macrophages after hydrogel administration or implantation. That is, polarization into anti-inflammatory M2 macrophages may be promoted.

In the present invention, the hydrogel may not cause hemolytic anemia or may not cause inflammatory bowel disease, drug cytotoxicity, or aplastic anemia. In addition, it may not cause kidney damage or decreased kidney functions, liver damage or decreased liver functions, or rapid increase or decrease in blood glucose.

As another aspect of the present invention, use of the hydrogel for implantation into muscle tissue; and use for preventing or treating muscle disease are provided.

As another aspect of the present invention, use of the hydrogel for preparing a composition for implantation into muscle tissue or a drug for preventing or treating muscle disease is provided.

In the present invention, the description regarding extracellular matrix, hydrogel, muscle disease, and the like are equally applied in relation to the use.

Provided is a method for preparing a hydrogel composition, the method comprising: cutting muscle tissue along the muscle grain; decellularizing the cut muscle tissue by treating it with 0.1% to 0.3% trypsin/ ethylenediaminetetraacetic acid (EDTA) for two to four hours, with 0.1% to 1.0% Triton X-100 for 10 to 14 hours, and with a mixed solution of 0.5% to 1.5% Triton X-100 and 0.1% to 0.3% sodium deoxycholate for 20 to 24 hours; modifying the decellularized MEM with at least one functional group selected from the group consisting of a catechol group and a pyrogallol group; and cross-linking between the modified extracellular matrices.

Hereinafter, one or more specific embodiments are described in more detail by way of examples. However, these examples are provided for describe one or more embodiments in an illustrative manner, and the scope of the present invention is not limited to these examples.

### Example 1. Development of hydrogel based on phenol group-modified muscle tissue-derived extracellular matrix (MEM) for muscle regeneration

Catechol group (CA)/pyrogallol group (PG)-modified MEM (MEM-CA, MEM-PG) derivatives were synthesized by modifying MEM that has undergone decellularization and solubilization with a catechol group and a pyrogallol group through a chemical reaction using 1-ethyl-3-(3-dimethylaminopropyl)carbodiimide (EDC)/N-hydroxysuccinimide (NHS).

The synthesized MEM-CA/PG derivatives were naturally oxidized in an environment where oxygen was present in the body, and cross-linking between the extracellular matrix derivatives was induced to form hydrogels.

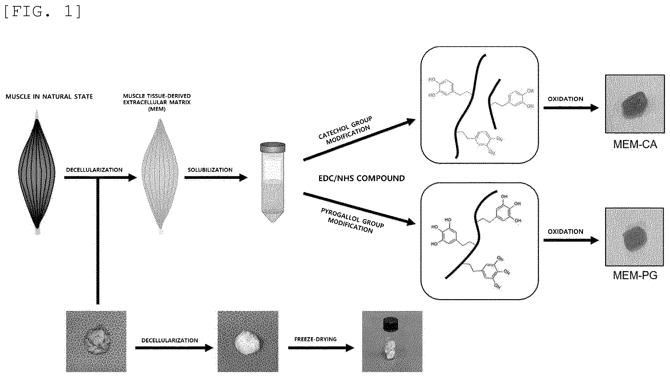

Therefore, it was confirmed that when the MEM-CA/PG solution is injected into the body, it reacts with oxygen in the body and so a hydrogel may be formed without a further cross-linking agent (FIG. 1).

### Example 2. Preparation of analysis of MEM

A porcine skeletal muscle tissue was cut along the muscle grain and decellularized by treating it with 0.2% EDTA for three hours, with 0.5% Triton X-100 for 12 hours, and with a mixed solution of 1% Triton X-100 and 0.2% sodium deoxycholate for 22 hours. Thereafter, decellularization and preservation of the extracellular matrix components were confirmed through histological staining and a quantitative analysis of DNA, glycosaminoglycan (GAG), and collagen.

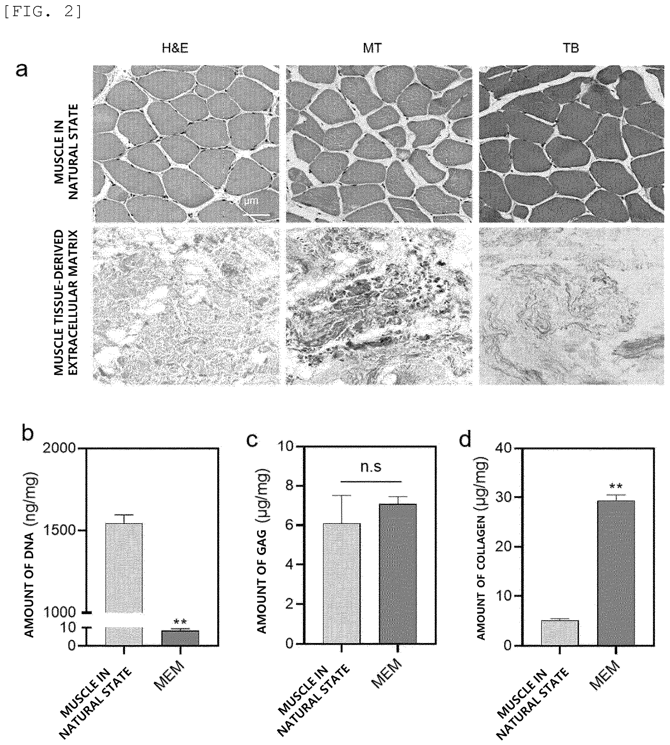

Through Hematoxylin & Eosin (H&E) staining, it was confirmed that cellular components (nuclei) were effectively removed from the MEM, and through Masson's trichrome (MT) histological staining, it was confirmed that collagen components were well preserved. A toluidine blue (TB) staining analysis was applied to confirm that GAG components were well preserved (FIG. 2A) .

To confirm whether the decellularization process was successful, the residual dsDNA in the MEM was quantitatively analyzed. It was confirmed that 8.53 ng DNA/mg tissue remained in the MEM. This showed that dsDNA was present in the MEM at a level less than 50 ng/mg, which is the criterion for a clinically applicable decellularized tissue, and 99.4% of the nuclei were removed compared to the existing muscle tissue (1544.18 ng/mg), confirming successful decellularization (FIGS. 2B).

A quantitative analysis was performed to confirm whether the major extracellular matrix components, GAG and collagen, were well preserved without damage after the decellularization process. Since only the extracellular matrix components remain after the decellularization process, it was confirmed the GAG and collagen contents were increased in the MEM compared to the muscle tissue before the decellularization. In the MEM, the GAG and collagen components were detected at 7.08 ug/mg and 29.4261 ug/mg, respectively, which were contained in greater amounts than the existing porcine muscle tissue (6.10 µg/mg and 5.21 ug/mg, respectively), thereby confirming that the extracellular matrix components were well preserved (FIGS. 2C and 2D).

Through these results, it was confirmed that the cells were successfully removed from the muscle tissue through the decellularization process of the present invention and that the extracellular matrix components were well preserved.

### Example 3. Proteomics analysis of extracellular matrix from decellularized muscle tissue

A quantitative proteomic analysis was performed on the MEM using high-resolution mass spectrometry.

The MEM was composed of 62.36% collagen and further contained various matrisome proteins such as 3.28% ECM glycoproteins, 34.24% proteoglycans, and 0.11% ECM regulators (FIG. 3A) .

Collagen is an extracellular matrix protein that occupies the most part in actual muscle, and when 10 matrix proteins most abundant in the MEM were investigated, it was confirmed that there were four collagen types. In particular, among them, collagen type 6 is known as one of the proteins that play an important role in maintaining muscle functions (FIG. 3B).

When a gene ontology (GO) analysis was performed on the matrisome proteins in the MEM, proteins related to extracellular matrix composition and structure, collagen composition, cell adhesion, and tissue development were mainly identified in the MEM, and thus it was expected that the MEM could contribute to muscle regeneration through muscle structure construction, development, etc. (FIG. 3C).

When a gene ontology (GO) analysis was performed on the non-matrisome proteins in the MEM, a number of proteins related to muscle structure development, muscle contractile functions, and metabolic functions were identified (FIG. 3D).

It was confirmed that various collagens, glycoproteins, and proteoglycans proteins were preserved in the MEM even after the decellularization process (FIG. 4A).

The porcine-derived MEM and the actual human muscle proteins were compared. It was confirmed that all 28 matrisome proteins and all 51 muscle tissue-specific proteins (expressed more than 4-fold compared to other tissues) of the MEM were included in the proteins constituting human muscle tissue. Therefore, since the MEM components are similar to the actual human muscle tissue, it was confirmed that the MEM may provide an actual muscle microenvironment (FIG. 4B).

Therefore, through this GO analysis and comparison of porcine-derived MEM and actual human muscle protein, it was confirmed that the MEM contributes to muscle development and functions and metabolic functions, and it may contribute to the promotion of muscle tissue regeneration, since it can provide a muscle-specific microenvironment.

### Example 4. Internal structure analysis of phenol group-modified MEM hydrogels

The internal structures of MEM, MEM-CA, and MEM-PG hydrogels were analyzed by scanning electron microscopy.

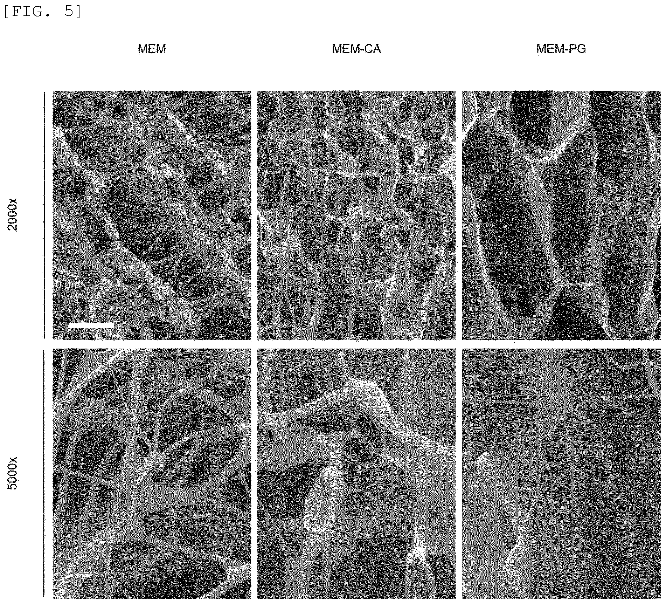

It was observed that the MEM hydrogel has a structure similar to the nanofibers observed inside the collagen hydrogel, and it was confirmed in the case of MEM-CA and MEM-PG hydrogels, these nanofibrous structures and micro-scale dense porous structures are present together (FIG. 5).

### Example 5. Chemical structure analysis of phenolic group-modified MEM hydrogels

Through a Fourier-transform infrared spectroscopy (FT-IR) analysis, the chemical structures of the MEM, MEM-CA, and MEM-PG solutions and each of the hydrogels were analyzed.

A peak at ~3300 cm⁻¹ (yellow bar) representing the -OH group was observed in all groups, and it was confirmed that the corresponding peak increased further in each hydrogel.

The amide I, amide II, and amide III peaks, which are mainly observed in extracellular matrix proteins, were observed in all groups at ~1650 cm⁻¹ (pink bar), ~1550 cm⁻¹ (orange bar), and ~1260 cm⁻¹ (green bar), respectively, and only in the hydrogel groups, it was observed that the amide II peak was decreased and the amide III peak was shifted from 1240 cm⁻¹ to 1260 cm⁻¹, confirming the structural change of the backbone during the conversion to a hydrogel.

Both the peak at ~1450 cm⁻¹ (brown bar) representing aromatic -OH stretching and the peak at ~800 cm⁻¹ (purple bar) representing a biphenol group were observed, and it was confirmed that the peak intensity increased further because the phenol groups were oxidized and dimerized in the hydrogels.

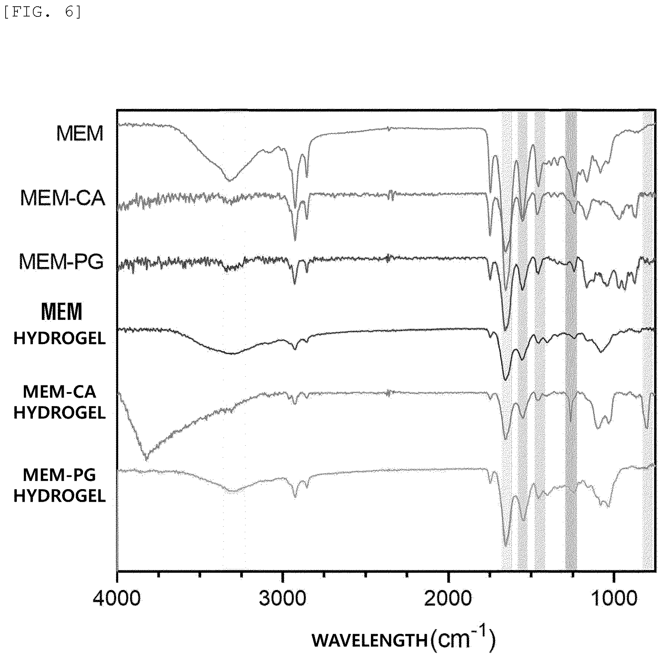

Therefore, it was confirmed that the MEM was modified with a catechol group and a pyrogallol group, and dimerization of the catechol groups or pyrogallol groups occurred during oxidation, resulting in cross-linking (FIG. 6).

### Example 6. Analysis of degradation pattern of phenol group-modified MEM hydrogels

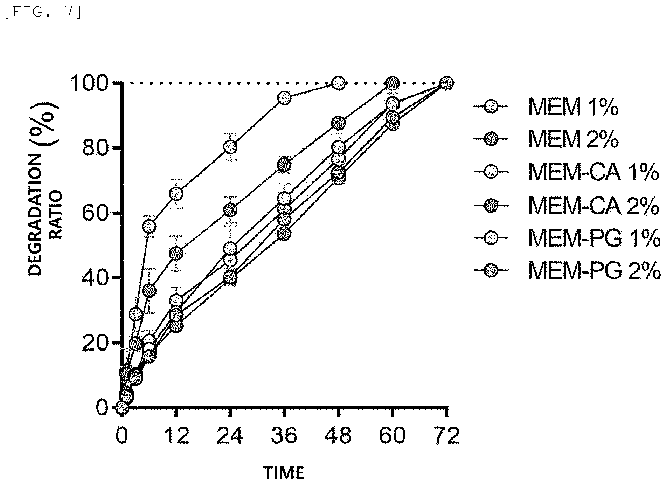

To compare the degradation pattern of the MEM, MEM-CA, and MEM-PG hydrogels under two concentration conditions of 1% and 2% (w/v), the hydrogels were treated with a collagenase (50 pg/ml), and it was confirmed that, compared to the MEM hydrogel, which was not modified with a phenol group, the MEM-CA and MEM-PG hydrogels were degraded slowly due to a cross-linking reaction between the catechol groups or pyrogallol groups (FIG. 7).

Therefore, it is predicted that the MEM-CA and MEM-PG hydrogels may be maintained more stably than the MEM hydrogel even under degradation conditions *in vivo.*

### Example 7. Analysis of mechanical properties of phenol group-modified MEM hydrogels

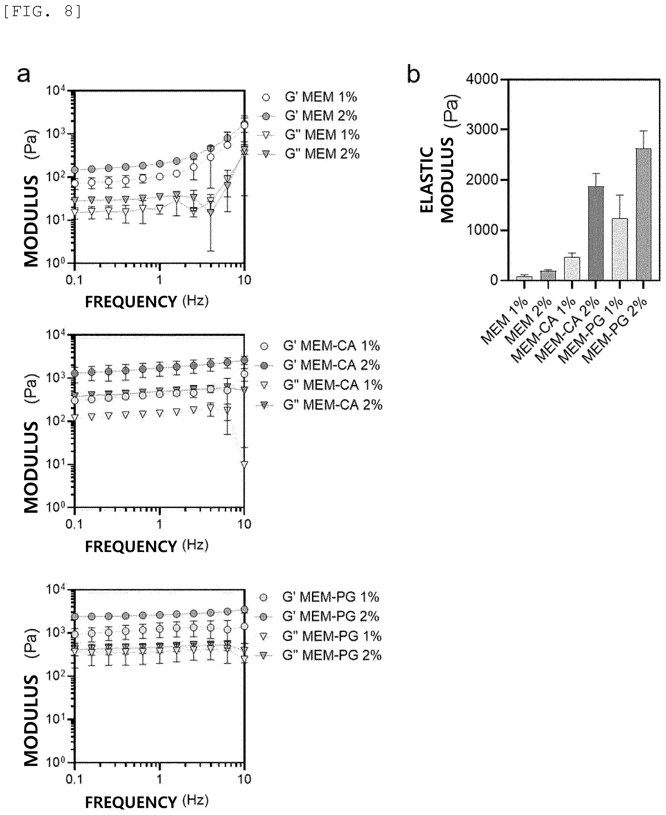

The elastic modulus of each of the MEM, MEM-CA, and MEM-PG hydrogels under two concentration conditions of 1% and 2% (w/v) was measured using rheological analysis equipment (FIGS. 8A and 8B). It was confirmed that the G' (storage modulus) value was higher than the G'' (loss modulus) value in all the hydrogels, confirming that a stable polymer network structure was formed inside all the hydrogels.

The average elastic modulus of 1% MEM and 2% MEM hydrogels were measured to be about 92 Pa and about 172 Pa, respectively, the average elastic modulus of 1% MEM-CA and 2% MEM-CA hydrogels were measured to be about 468 Pa and about 1800 Pa, respectively, and the average elastic modulus of 1% MEM-PG and 2% MEM-PG hydrogels were measured to be about 1240 Pa and about 2628 Pa, respectively, and thus it was confirmed that the elastic modulus increased in proportion to the concentration of each hydrogel, and the MEM hydrogels modified with a catechol group or pyrogallol group had a much increased elastic modulus compared to the MEM hydrogels (FIG. 8B). In addition, it could be seen that the measured elastic modulus of the MEM-PG hydrogels was higher than that of the MEM-CA hydrogels at the same concentration.

Through these analyzes, it was confirmed that the mechanical properties of the MEM hydrogels may be controlled through concentration change and chemical modification, and as a result, it was predicted that based on their improved physical and mechanical properties, the phenol group-modified MEM hydrogels may provide a muscle tissue microenvironment and promote muscle regeneration.

### Example 8. Injection force analysis of phenol group-modified MEM hydrogels

In order to compare the injection force of each of the MEM, MEM-CA, and MEM-PG hydrogels under two concentration conditions of 1% and 2% (w/v), each of MEM, MEM-CA, and MEM-PG in their solution state was added into a 26-gauge (1 ml) syringe, and the force required for injection was measured while pressing the pistol at a speed of 30 mm/min.

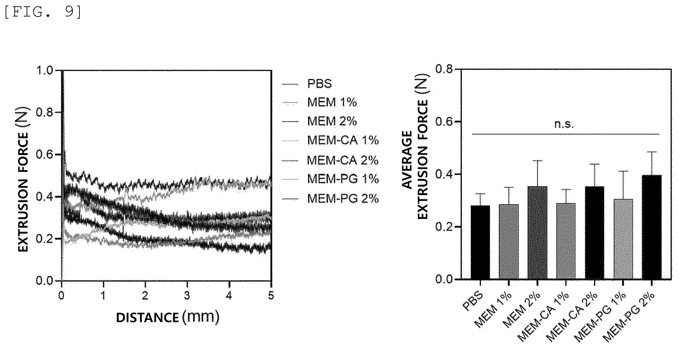

It was confirmed that there was not a significant difference in all the groups compared to the force required when injecting phosphate buffered saline (PBS), confirming that the MEM-CA and MEM-PG hydrogels may be easily injected through a syringe and are easy to use (FIG. 9).

### Example 9. Analysis of antioxidant function of phenol group-modified MEM hydrogels

The MEM-CA and MEM-PG hydrogels were predicted to have the ability to scavenge reactive oxygen species (ROS) and various radicals due to the oxidized derivatives of the modified phenol groups.

Therefore, by measuring the hydroxyl radical scavenging ability and 2,2'-azino-bis(3-ethylbenzothiazoline-6-sulfonic acid (ABTS) radical scavenging ability, the antioxidant effect of each of the MEM, MEM-CA, and MEM-PG hydrogels was compared. Ascorbic acid, known as vitamin C, with excellent antioxidant function was used as a control group.

A Fenton reaction reagent in which H₂O₂ and Fe²⁺ ions react to generate Fe³⁺ ions and hydroxyl radicals which induce DNA damage and cell death was used to confirm whether the reaction was inhibited by MEM-CA and MEM-PG. In addition, using a method based on the ABTS solution, which is one of the most widely used anti-oxidation test methods, the degree to which the blue-green ABTS solution becomes transparent when reacted with a sample having an antioxidant function was compared.

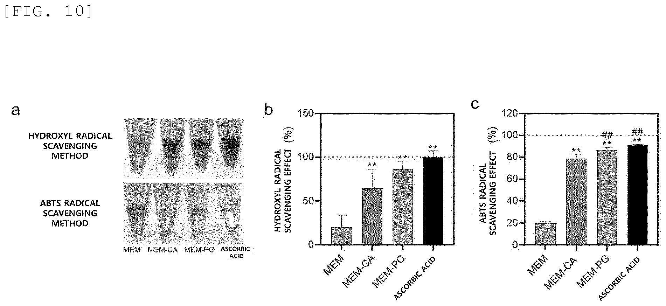

2% (w/v) MEM-CA and MEM-PG hydrogels were added to a solution prepared by using the Fenton reaction and the ABTS solution and the incubated for 30 minutes, and then the color of the solutions was measured by UV-vis spectroscopy to confirm whether the hydroxyl radicals and ABTS radicals were scavenged, respectively. As a result, it was confirmed that MEM-CA and MEM-PG hydrogels modified with a catechol group and a pyrogallol group had an antioxidant function similar to that of ascorbic acid, a positive control group. Therefore, it was predicted that in the treatment of senescent sarcopenia and muscle damage facilitated by ROS and free radicals, the MEM-CA and MEM-PG hydrogels may effectively remove these harmful substances, thereby promoting effective muscle regeneration compared to unmodified MEM hydrogels (FIG. 10).

### Example 10. Immunogenicity evaluation of phenol group-modified MEM hydrogels

While co-cultivating macrophages and hydrogels for 24 hours, the amount of pro-inflammatory cytokines secreted from macrophages, tumor necrosis factor alpha (TNF-α) and interleukin-6 (IL-6), was measured through enzyme-linked immunosorbent assay (ELISA) to evaluate the immunogenicity level of the hydrogels.

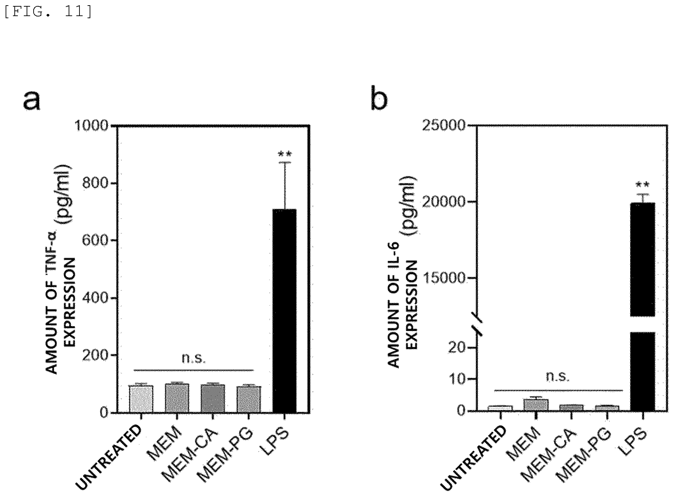

When co-cultured with 2% (w/v) MEM, MEM-CA, and MEM-PG hydrogels, the amount of TNF-α secretion from macrophages was 101 pg/ml, 97 pg/ml, and 92 pg/ml, respectively, and so it was confirmed that there was almost no difference compared to the amount of TNF-α secretion of 96 pg/ml in the control group (no treatment) without any treatment. On the other hand, it was confirmed that in the positive control group treated with lipopolysaccharides(LPS), a substance that induces an immune response, the amount of TNF-α secretion was 711 pg/ml, indicating that the expression and secretion of TNF-α were greatly increased (FIG. 11A).

When co-cultured with 2% (w/v) MEM, MEM-CA, and MEM-PG hydrogels, the amount of IL-6 secretion from macrophages was 3.7 pg/ml, 1.8 pg/ml, and 1.7 pg/ml, respectively, and so it was confirmed that there was almost no difference compared to the amount of IL-6 secretion of 1.5 pg/ml in the control group (no treatment) without any treatment.

On the other hand, it was confirmed that in the positive control group treated with LPS, the amount of IL-6 secretion was 19918.1 pg/ml, indicating that the expression and secretion of IL-6 were greatly increased (FIG. 11B).

Therefore, it could be seen that the MEM-CA and MEM-PG hydrogels have a very low possibility of inducing an immune response and have excellent biocompatibility.

### Example 11. Evaluation of biocompatibility of phenol group-modified MEM hydrogels

On days 3, 7, and 14 after injecting PBS and 2% (w/v) MEM, MEM-CA, and MEM-PG hydrogels to the mouse tibialis anterior (TA) muscle in a volume of 50 µl, the TA muscle tissue was extracted and subjected to a histological analysis performed through H&E staining and TB staining. The above experiment was conducted to confirm tissue damage and immune response that may occur when a hydrogel enters into a tissue.

In addition, to evaluate the biocompatibility of MEM-CA and MEM-PG hydrogels, on days 3, 7, and 14 after intramuscularly injecting 2% (w/v) hydrogel into the mouse TA muscle in a volume of 50 µl, the TA muscle tissue was extracted, and immunohistochemical staining was performed.

To evaluate the biocompatibility of MEM-CA and MEM-PG hydrogels, on days 3, 7, and 14 after intramuscularly injecting 2% (w/v) hydrogel into the mouse TA muscle in a volume of 50 µl, blood was extracted and analyzed. This was to confirm the inflammatory response that occurs in the body when a hydrogel is injected into a tissue was conducted to confirm the degree of expression of macrophages in the hydrogel through the inflammatory marker M1 macrophage marker (iNOS) and the anti-inflammatory marker M2 macrophage marker (CD206).

To confirm the inflammatory response following hydrogel injection, the white blood cell (WBC) and lymphocyte levels were confirmed, and to confirm the causing of hemolytic anemia, which may be caused by toxic substances, drugs, infections, kidney abnormalities, and liver abnormalities, red blood cell (RBC), hemoglobin (HGB), hematocrit (HCT), mean corpuscular volume (MCV), mean corpuscular hemoglobin (MCH), and mean corpuscular hemoglobin concentration (MCHC) values were confirmed.

In addition, the mean platelet volume (MPV) value is an indicator of various diseases, and its high values are indicative of heart disease, hypertension, and hyperthyroidism, and its low values are indicative of inflammatory bowel disease, drug cytotoxicity, and aplastic anemia.

To determine whether the kidney functions are affected, a kidney function test was performed using serum samples. Blood urea nitrogen (BUN) and creatinine (CRE) concentrations were confirmed as indicators of kidney functions, and aspartate transaminase (AST), alanine transaminase(ALT), and total protein (TP) were used as indicators to test the degree of liver damage and functions. The above experiment was conducted to confirm the safety of the hydrogel based on the inflammatory changes that may occur when a substance outside a living organism is injected into the tissue.

On days 3, 7, and 14 after injecting PBS and 2% (w/v) MEM, MEM-CA, and MEM-PG hydrogels to the mouse TA muscle in a volume of 50 µl, the major organs of the mouse (heart, liver, spleen, lungs, kidneys) were extracted and subjected to a histological analysis performed through H&E staining. The experiment was conducted to confirm the safety of the implanted hydrogel to the major organs.

### Example 12. Verification of muscle mass increasing effect by phenol group-modified MEM hydrogels in disuse muscle atrophy animal model

The MEM, MEM-CA, and MEM-PG hydrogels were applied to a disuse muscle atrophy small animal model (mouse model of disuse muscle atrophy) to evaluate the effect of increasing muscle mass. After fixing a mouse's leg with surgical staples for three weeks to induce disuse skeletal muscle atrophy, which reduced the weight of the TA muscle by about 25%, each of the 2% (w/v) hydrogels was intramuscularly injected to the TA muscle in a volume of 50 µl, and then the TA muscle tissue was extracted in week 2 to compare the weight with the normal tissue (the leg muscle on the opposite side of the same subject used without causing any damage).

### Example 13. Comparison of muscle mass increasing effect between phenol group-modified MEM hydrogels and various hydrogels developed in previous research in disuse muscular atrophy animal model

Phenol-modified muscle tissue extracellular matrix hydrogels MEM-CA and MEM-PG, phenol-modified skin tissue extracellular matrix hydrogels Skin-CA and Skin-PG, and HA-CA/MEM and HA-PG/MEM hydrogels, which were prepared by mixing a phenol group-modified hyaluronic acid developed through previous research with a muscle tissue extracellular matrix, were applied to a disuse muscle atrophy small animal model (mouse model of disuse muscle atrophy) to compare the muscle mass increasing effect. After fixing a mouse's leg with surgical staples for three weeks to induce disuse skeletal muscle atrophy, which reduced the weight of the TA muscle by about 25%, each of the 2% (w/v) hydrogels was intramuscularly injected to the TA muscle in a volume of 50 µl, and then the TA muscle tissue was extracted in week 2 to compare the weight with the normal tissue.

### Example 14. Proteomics analysis and comparison of decellularized MEM and decellularized skin tissue-derived extracellular matrix

To confirm that the MEM promotes tissue-specific muscle regeneration, the proteomes of MEM and the SkEM were compared.

### Example 15. Verification of muscle mass increasing effect of phenol group-modified MEM hydrogels in sarcopenia animal model

The MEM, MEM-CA, and MEM-PG hydrogels were injected into a sarcopenia small animal model (mouse model of sarcopenia) and the muscle mass increasing effect was evaluated. After intramuscularly injecting each of the 2% (w/v) hydrogels into the TA muscle of 18-month-old aging mice in a volume of 50 µl, the TA muscle tissue was extracted at week 8, and the muscle weight was compared with the mouse weight.

### Example 16. Evaluation of biocompatibility of phenol group-modified MEM hydrogels in sarcopenia animal model

PBS and MEM, MEM-CA, and MEM-PG hydrogels were applied to a mouse model of sarcopenia, and the biocompatibility was evaluated. After intramuscularly injecting each of 2% (w/v) hydrogels into the TA muscle of 18-month-old aging mice in a volume of 50 µl, major organs (heart, liver, spleen, kidneys, lungs) of the mice were extracted at week 8, and a histological analysis was performed through H&E staining.

### Experimental Example 1. Results of evaluating biocompatibility of phenol group-modified MEM hydrogels

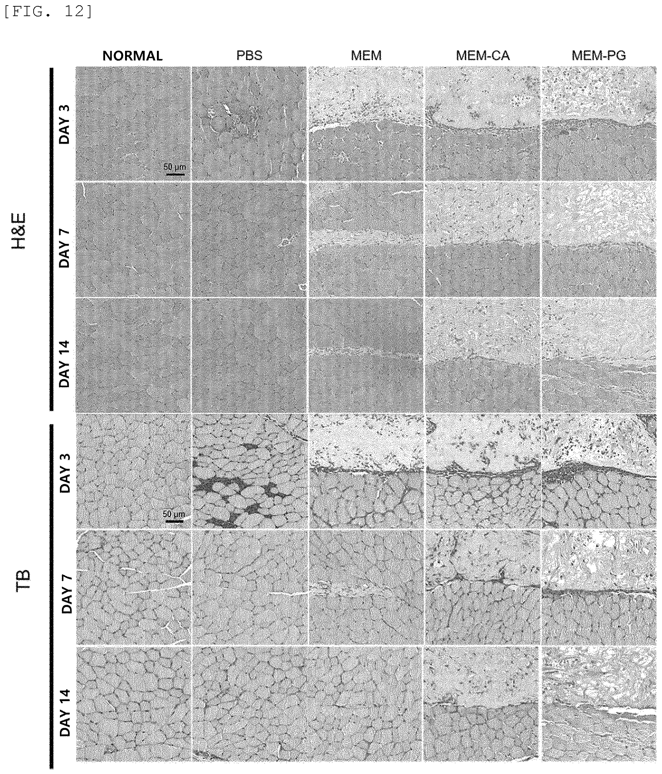

As an experiment to confirm biocompatibility, on days 3, 7, and 14 after injecting PBS and 2% (w/v) MEM, MEM-CA, and MEM-PG hydrogels into the mouse TA muscle in a volume of 50 µl, the TA muscle tissue was extracted and subjected to a histological analysis performed through H&E staining and TB staining. The results confirmed that, compared to the normal group (normal tissue) that was not subjected to any treatment, in the group injected with PBS and each hydrogel, cells migrated to and distributed in the region where the foreign substance was injected on day 3 after injection, but most of these cells disappeared on day 14 after injection. When the presence of mast cells, a type of white blood cell, was confirmed through TB staining among the cells observed around the injected hydrogel, it was confirmed that there were almost no such cells. Therefore, it was confirmed that almost no tissue damage or immune response occurred due to the hydrogel injection (FIG. 12).

To evaluate the biocompatibility of the MEM-CA and MEM-PG hydrogels, on days 3, 7, and 14 after intramuscularly injecting 2% (w/v) hydrogel to the mouse TA muscle in a volume of 50 µl, the TA muscle tissue was extracted and subjected to immunohistochemical staining.

Thereafter, an inflammatory marker, M1 macrophage marker (iNOS), and an anti-inflammatory marker, M2 macrophage marker (CD206), were stained. As a result, it was confirmed that the expression of M1 macrophages increased on day 3 after the hydrogel injection and gradually decreased on days 7 and 14. On the contrary, it was confirmed that the expression level of M2 macrophages was found to be low on day 3, but it increased on days 7 and 14. In the group injected with PBS, it was confirmed that the expression of M1 macrophages increased on day 3 and decreased on day 7, and the expression of M2 macrophages was higher on day 7 than on day 3.

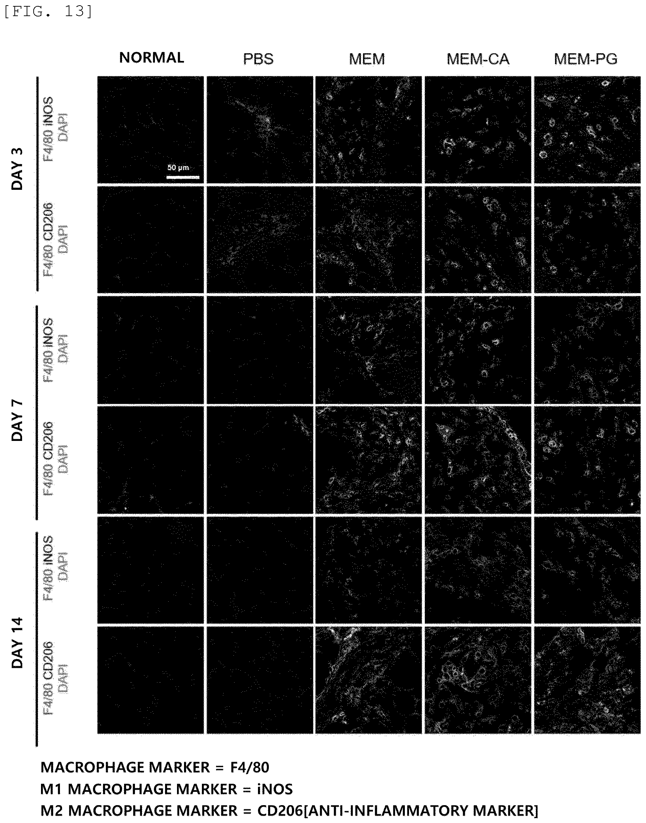

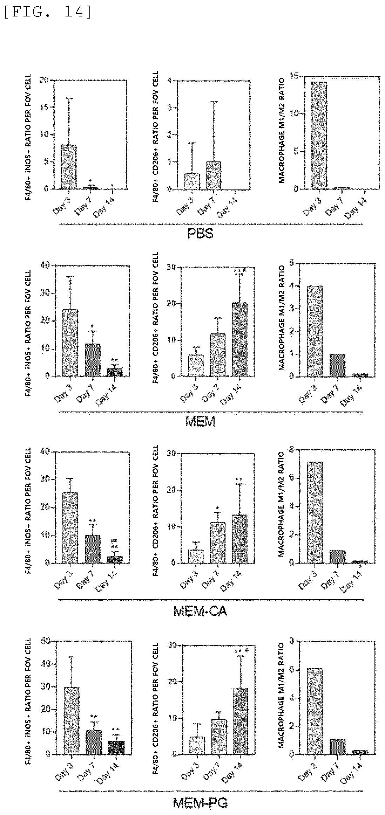

When the ratio of expressed M1/M2 macrophages was analyzed, it was confirmed that the proportion of M1 macrophages was higher on day 3 after the hydrogel injection, but the proportion of M2 macrophages was much higher on day 14. These results showed that after injection of MEM-CA and MEM-PG hydrogels, the expression of M2 macrophages increased more than that of M1 macrophages, thereby inducing an anti-inflammatory response rather than an inflammatory response (FIGS. 13 and 14).

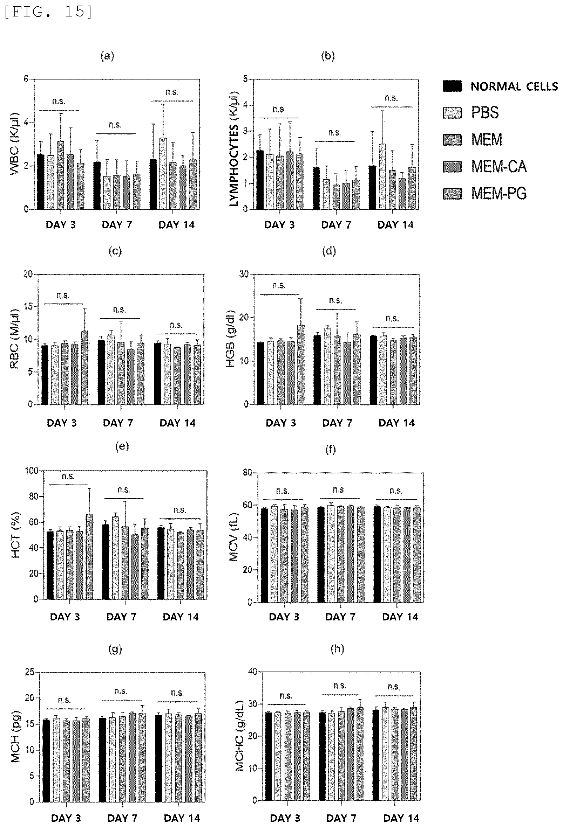

In addition, the levels of WBC and lymphocytes were confirmed to confirm the inflammatory response. It was confirmed that on days 3, 7, and 14, all the groups injected with the hydrogel exhibited values that were similar to those of the normal group (normal tissue) that was not subjected to any treatment, and there was no inflammatory reaction (FIGS. 15A and 15B).

To confirm whether the injected hydrogels cause hemolytic anemia, which may be caused by toxic substances, drugs, infections, kidney abnormalities, and liver abnormalities, the RBC, HGB, HCT, MCV, MCH) and MCHC values were confirmed. As a result, it was confirmed that on days 3, 7, and 14, all the groups injected with the hydrogel exhibited values that were similar to those of the normal group (normal tissue) that was not subjected to any treatment (FIGS.15C to 15H).

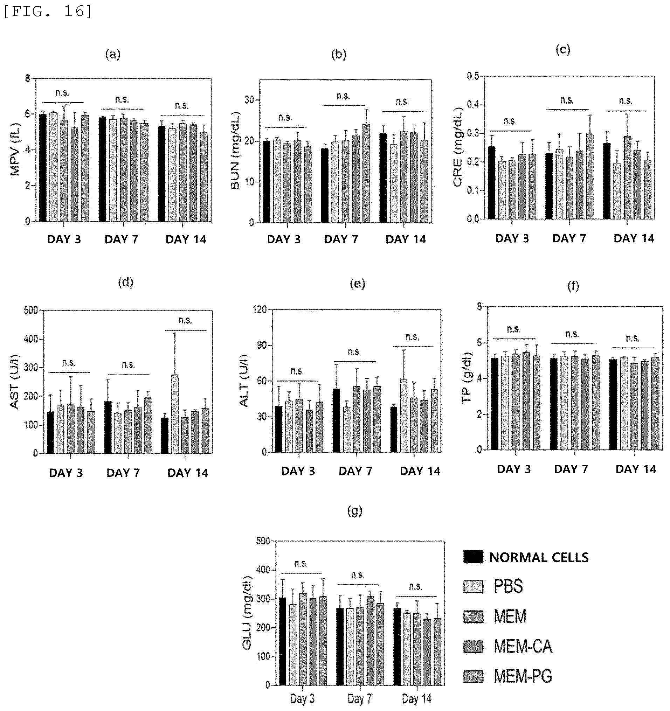

The mean platelet volume (MPV) value is an indicator of various diseases, and its high values are indicative of heart disease, hypertension, and hyperthyroidism, and its low values are indicative of inflammatory bowel disease, drug cytotoxicity, and aplastic anemia. When the MPV level was compared on days 3, 7, and 14, all the groups injected with the hydrogel exhibited a value that was similar to that of the normal group (normal tissue) that was not subjected to any treatment, confirming that the hydrogel injection did not cause disease or toxicity (FIG. 16A).

To determine whether the kidney functions are affected, a kidney function test was performed using serum samples. When the BUN and CRE concentrations were confirmed on days 3, 7, and 14, there was not a significant difference between any of the hydrogel groups and the normal group (normal tissue) that was not subjected to any treatment, confirming that the kidney functions were not affected (FIGS. 16B and 16C).

AST, ALT, and TP were used as indicators to test the degree of liver damage and functions. When liver damage occurs, the ALT and AST levels increase and the TP level decrease, and it was confirmed that on days 3, 7, and 14 after the hydrogel injection, the levels of all the groups were similar to those of the normal group (normal tissue) that was not subjected to any treatment. Therefore, it was confirmed that there was no liver damage caused by the hydrogel injection and that the liver functions were not affected (FIGS. 16D, 16E, and 16F).

Blood glucose (GLU, glucose) was measured because glucose in the blood increases when inflammation occurs. The blood glucose level in all the hydrogel groups was also similar to that of the normal group (normal tissue) that was not subjected to any treatment, and thus it was confirmed that no inflammation occurred (FIG. 16G).

According to the above experimental results, it was confirmed that when the hydrogels prepared in the present invention were injected, a mild immune response occurred initially and then gradually decreased, indicating that the MEM-CA and MEM-PG hydrogels are very unlikely to cause a serious inflammatory response. In addition, it was expected that the hydrogels could induce a muscle regeneration treatment effect by promoting polarization into anti-inflammatory macrophages that are helpful in tissue regeneration.

Therefore, through a series of comprehensive experiments, it was found that the MEM-CA and MEM-PG hydrogels have excellent biocompatibility.

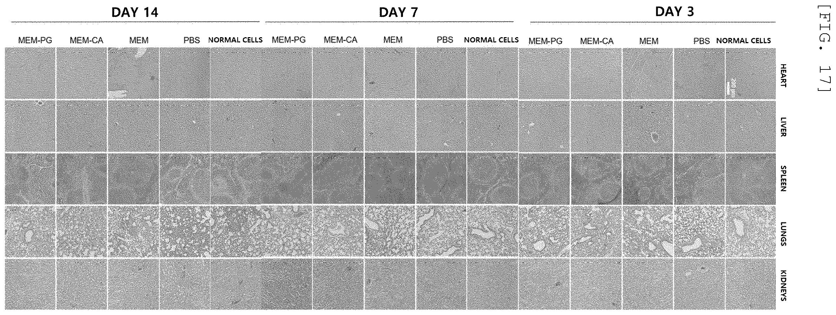

Finally, on days 3, 7, and 14 after injecting PBS and 2% (w/v) MEM, MEM-CA, and MEM-PG hydrogels to the mouse TA muscle in a volume of 50 µl, the major organs of the mouse (heart, liver, spleen, lungs, kidneys) were removed and subjected to a histological analysis performed through H&E staining. As a result, it was confirmed that no tissue damage or immune response occurred in the group injected with PBS or each hydrogel, as in the normal group (normal tissue) that was not subjected to any treatment (FIG. 17).

Therefore, the above-described results show that the hydrogel of the present invention may serve as an extracellular matrix for muscle regeneration.

### Experimental Example 2. Results of verifying muscle mass increasing effect of phenol group-modified MEM hydrogels in disuse muscular atrophy animal model

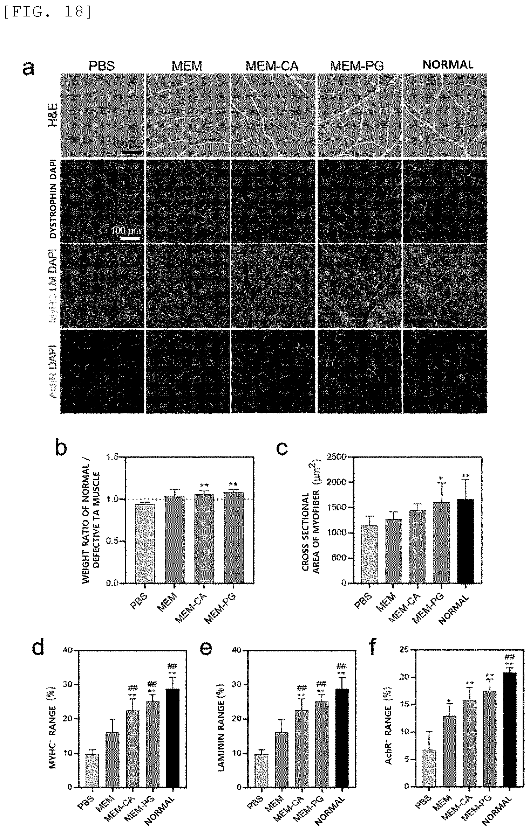

Through H&E staining and immunohistochemical staining, it was confirmed that the size of myofibers increased in the groups injected with the MEM-CA and MEM-PG hydrogels (FIGS. 18A and 18C).

In the groups injected with the MEM-CA and MEM-PG hydrogels, a muscle mass increasing effect to the level of normal tissue was observed, and it was confirmed that, compared to the non-treated group intramuscularly injected with PBS, there was a statistically significant effect of increasing leg muscle weight (FIG. 18B).

In addition, compared to the case of injecting the MEM hydrogel, the area of myosin heavy chain (MyHC)+ myofibers and the expression of acetylcholine receptor, which induces skeletal muscle contractile function, increased when the MEM-CA and MEM-PG hydrogels were injected, and successful induction of functional muscle mass increase was confirmed by confirming an increase in correct laminin deposition (FIGS. 18D, 18E, and 18F).

### Experimental Example 3. Results of comparing muscle mass increasing effect of phenol group-modified MEM hydrogels and various hydrogels developed in previous research in disuse muscular atrophy animal model

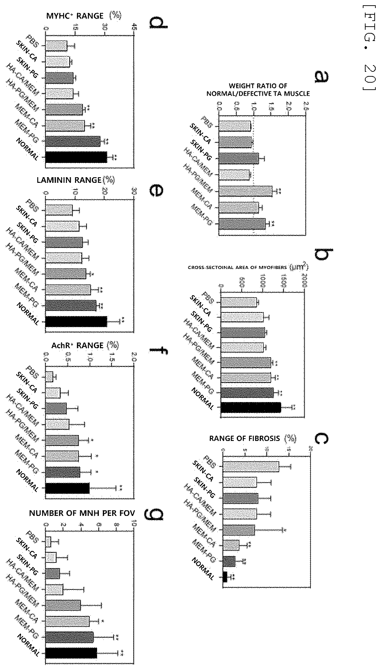

A muscle mass increasing effect was actually observed in the groups injected with each of MEM-PG and HA-PG/MEM, and it was confirmed that, compared to the non-treated group intramuscularly injected with PBS, there was a statistically significant effect of increasing leg muscle weight (FIG. 20A).

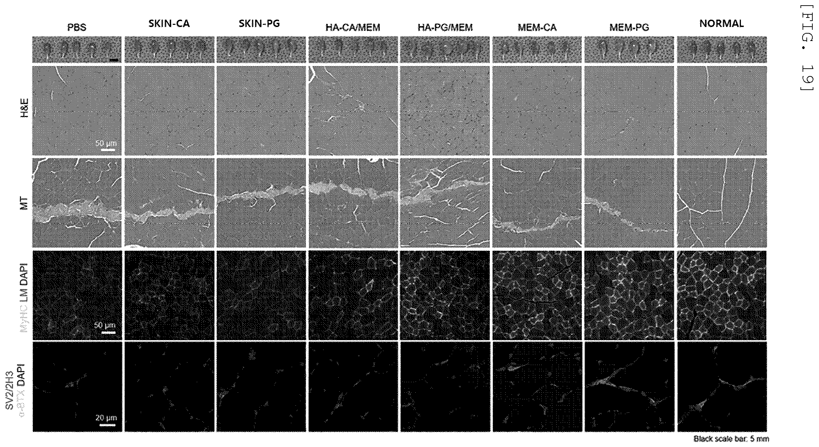

Through H&E staining and immunohistochemical staining, it was confirmed that the size of myofibers increased in the groups injected with the HA-PG/MEM, MEM-CA, and MEM-PG hydrogels (FIGS. 19 and 20B).

Through MT staining, it was confirmed that when each of the MEM-CA and MEM-PG hydrogels was injected, the area of muscle fibrosis was most significantly reduced compared to the case where PBS was injected (FIGS. 19 and 20C).

It was confirmed that, compared to the groups injected with each of PBS, Skin-CA, Skin-PG, and HA-CA/MEM, an increase in the area of MyHC myofibers and correct laminin (LM) deposition was confirmed in the groups injected with each of the HA-PG/MEM, MEM-CA, and MEM-PG hydrogels (FIGS. 20D and 20E) . In addition, through the increased expression of neuromuscular junction (NMJ) markers (SV2 (synaptic vesicle)/2H3 (neurofilament)), which induce skeletal muscle contractile function, and the detection after treatment with α-bungarotoxin (BTX), which is known to well bind to the α-subunit of the nicotinic acetylcholine receptor (AchR) of the neuromuscular junction (NMJ), it was confirmed that an increase of the functional muscle mass was successfully induced (FIGS. 20F and 20G).

Therefore, it was confirmed that the MEM-based hydrogels promote muscle regeneration in a tissue-specific manner. In addition, it was confirmed that applying a hydrogel formed by directly modifying MEM with a phenol group, as in the present invention, is more effective than simply mixing MEM with a hydrogel (HA-PG) developed through previous research and applying the resulting mixture.

### Experimental Example 4. Results of proteomics analysis and comparison of decellularized MEM and decellularized skin tissue-derived extracellular matrix

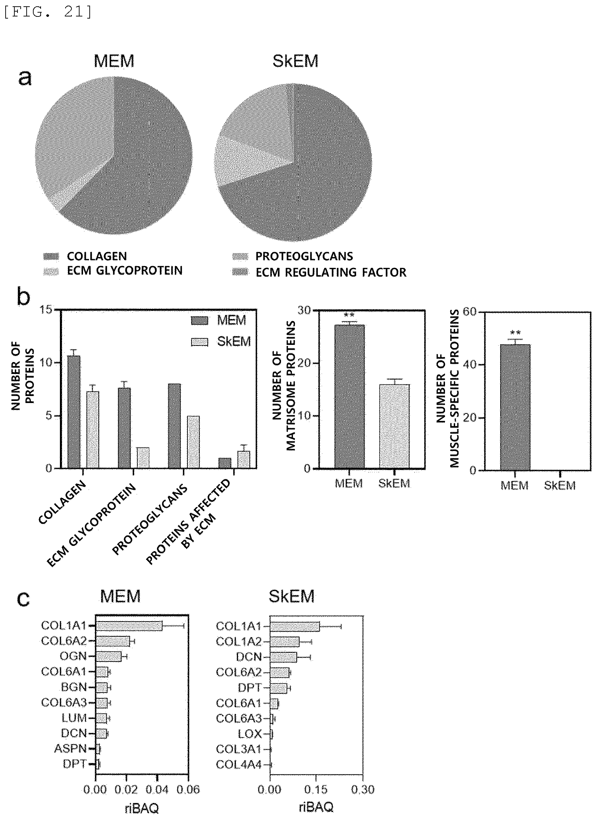

When the proteomes of MEM and SkEM were compared, it was confirmed that the protein components are different in general (FIG. 21A).

As a result of a matrisome protein analysis of MEM and SkEM, 27 proteins were detected in the MEM and 16 proteins in the SkEM, respectively. As a result of comparing the number of types (collagen, ECM glycoproteins, proteoglycans, ECM regulators), it was confirmed that MEM is composed of more diverse matrisome proteins (FIG. 21B). In particular, it was confirmed that MEM contains more diverse types of collagen and ECM glycoprotein proteins than SkEM.

In addition, an average of 47 muscle tissue-specific proteins (proteins reported to be expressed four times more in muscle tissue than other tissues) were detected in MEM, whereas no muscle tissue-specific proteins were detected in SkEM. Therefore, it was confirmed that MEM may better mimic an extracellular matrix microenvironment in an actual muscle tissue compared to SkEM (FIG. 21B).

When the 10 major matrisome proteins most abundantly detected in MEM and SkEM were compared, it was confirmed that only four proteins were the same and the remaining six proteins were different (FIG. 21C).

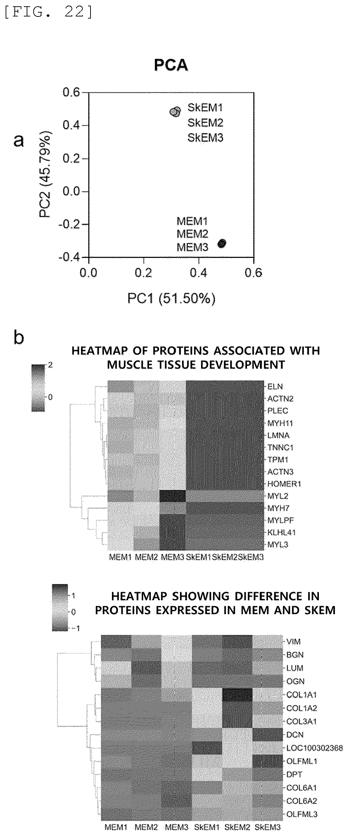

As a result of conducting a PCA on the total proteins of MEM and SkEM, there was a significant distance in the distribution of MEM and SkEM, indicating a clear difference in the components between the two matrices (FIG. 22A).

When MEM and SkEM were compared for proteins related to muscle tissue development in a gene ontology analysis, it was confirmed that there were a large number of proteins related to muscle development and their expression levels were high in MEM, whereas the proteins were almost absent or exhibited a significantly low expression level in SkEM (FIG. 22B).

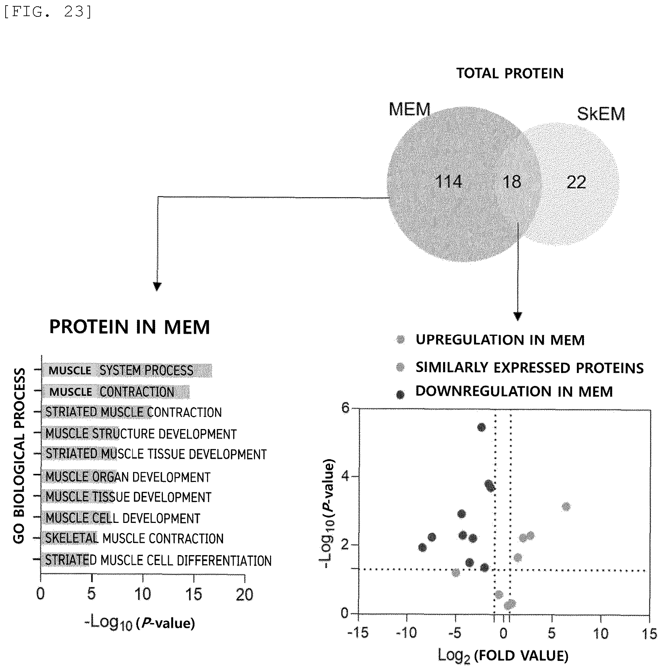

As a result of comparing the total protein composition of MEM and SkEM, it was confirmed that 18 proteins were commonly contained in the two tissue-derived matrices. As a result of comparing the expression levels of the 18 common proteins, 14 proteins showed different expression patterns. Among these, 4 proteins were expressed relatively higher in MEM than in SkEM, and 10 proteins had relatively low expression levels. This shows that the protein expression patterns of MEM and SkEM are different even though they contain the same protein. Therefore, it was confirmed that the types and contents of the components constituting the proteome of each matrix are different. In addition, as a result of a gene ontology analysis of proteins expressed only in muscles, it was confirmed that MEM consists of proteins related to muscle functions and development. These differences may be expected to contribute to the muscle developing and regenerating effects of MEM (FIG. 23).

### Experimental Example 5. Results of verifying muscle mass increasing effect of phenol group-modified MEM hydrogels in sarcopenia animal model

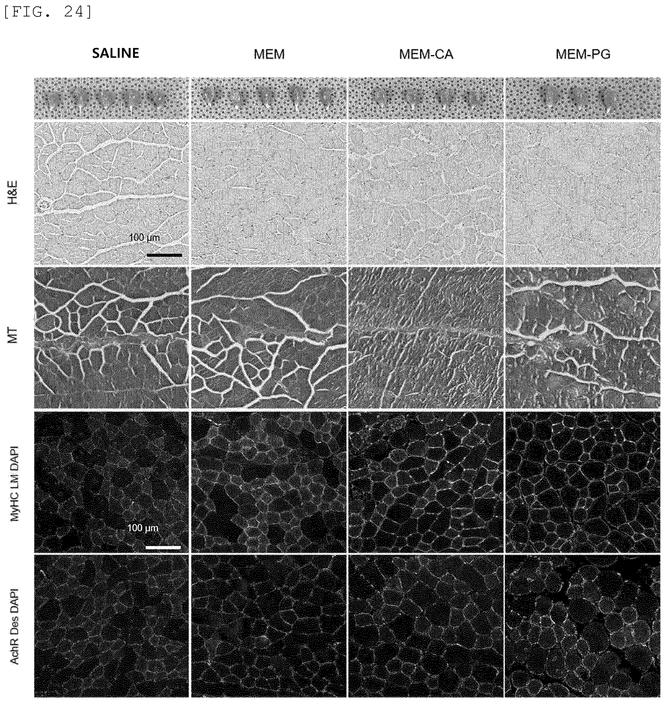

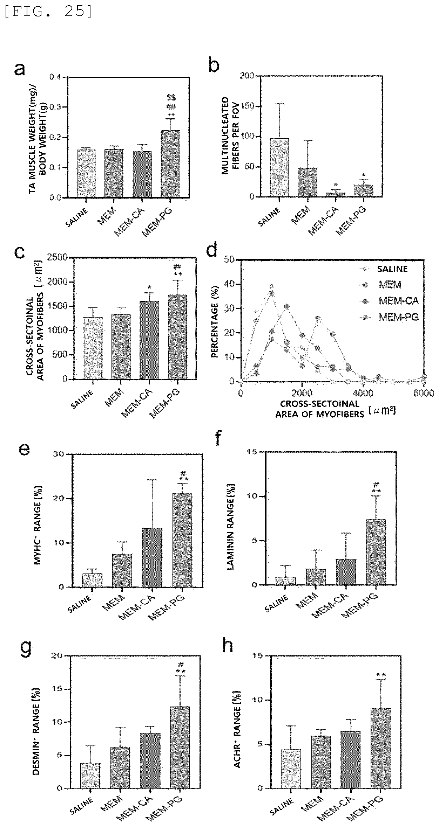

As a result of verifying a muscle mass increasing effect, a muscle mass increasing effect to the level of normal tissue was observed in the group injected with MEM-PG, and it was confirmed that, compared to the non-treated group intramuscularly injected with saline, there was a statistically significant effect of increasing leg muscle weight (FIGS. 24 and 25A). In addition, it was confirmed that the number of multinucleated muscle fibers positioned at the center of the muscle bundle was reduced in the groups injected with each of the MEM-CA and MEM-PG hydrogels, which is a result showing that the disease patterns commonly observed in sarcopenia muscle bundles were reduced, thus indicating that the injected MEM-CA and MEM-PG hydrogels had a treatment effect (FIGS. 24 and 25B).

Through H&E staining, MT staining, and immunohistochemical staining, it was confirmed that the size of myofibers increased in the groups injected with each of the MEM-CA and MEM-PG hydrogels (FIGS. 24, 25C, and 25D). In addition, it was confirmed that when each of the MEM-CA and MEM-PG hydrogels was injected, the MyHC+ myofiber area, correct LM deposition area, Desmin+ area, and the expression of acetylcholine receptor (AchR), which is responsible for skeletal muscle contractile function, were increased compared to the case where the MEM hydrogel was injected, confirming that an increase of functional muscle mass was successfully induced (FIGS. 24, 25E, 25F, 25G, and 25H).

### Experimental Example 6. Results of evaluating biocompatibility of phenol group-modified MEM hydrogels in sarcopenia animal model

PBS and MEM, MEM-CA, and MEM-PG hydrogels were applied to the TA muscle of 18-month-old aging mice. In week 8 after intramuscularly injecting the hydrogel to each in a volume of 50 µl, and major organs (heart, liver, spleen, kidneys, lungs) of the mice were extracted at week 8, and a histological analysis was performed through H&E staining.

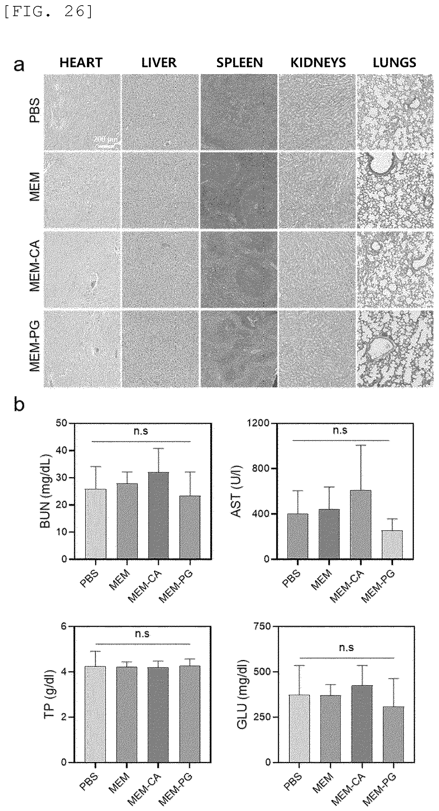

As a result, it was confirmed that no tissue damage or immune response occurred in major organs in the groups injected with PBS or hydrogel, as in the Normal group (normal tissue) (FIG. 26A) .

In addition, blood from the mice was extracted and analyzed. When the kidney function indicator BUN, the liver function indicator AST, total protein (TP), and blood glucose(GLU) levels were confirmed, it was confirmed that there was no significant difference in the groups injected with the MEM-CA and MEM-PG hydrogels compared to the group injected with PBS (FIG. 26B) .

Therefore, by confirming that no toxicity or immune response occurred even eight weeks after the hydrogel injection, it was confirmed that the MEM-CA and MEM-PG hydrogels have no problems with long-term application in the body and have excellent biocompatibility.

So far, the present invention has been examined focusing on its preferred embodiments. One of ordinary skill in the art to which the present invention pertains will understand that the present invention may be implemented in a modified form without departing from the essential characteristics of the present invention. Therefore, the disclosed embodiments should be considered from an illustrative perspective rather than a restrictive perspective. The scope of the present invention is indicated in the claims rather than the foregoing description, and all differences within the equivalent scope should be construed as being included in the present invention.

## Claims

1. A hydrogel composition comprising a decellularized muscle tissue-derived extracellular matrix (MEM) modified with one or more selected from the group consisting of a catechol group and a pyrogallol group.

2. The hydrogel composition according to claim 1, wherein in the composition, the hydrogel is formed by cross-linking one or more selected from the group consisting of a catechol group and a pyrogallol group with which an extracellular matrix is modified.

3. The hydrogel composition according to claim 1, wherein in the hydrogel composition, when the extracellular matrix is modified with a catechol group, a catechol group-modified decellularized MEM (MEM-CA derivative) is contained in an amount of 0.1% to 4% by weight compared to the total volume of the hydrogel composition.

4. The hydrogel composition according to claim 1, wherein in the hydrogel composition, when the extracellular matrix is modified with a pyrogallol group, a pyrogallol group-modified decellularized MEM (MEM-PG derivative) is contained in an amount of 0.1% to 4% by weight compared to the total volume of the hydrogel composition.

5. The hydrogel composition according to claim 1, wherein the hydrogel composition has a G' (storage modulus) higher than G'' (loss modulus).

6. The hydrogel composition according to claim 1, wherein, when the decellularized MEM is modified with a catechol group and is contained in an amount of 0.1% to 4% by weight compared to the total volume of the hydrogel composition, the hydrogel composition has an elastic modulus of 400 to 2000 Pa.

7. The hydrogel composition according to claim 1, wherein, when the decellularized MEM is modified with a pyrogallol group and is contained in an amount of 0.1% to 4% by weight compared to the total volume of the hydrogel composition, the hydrogel composition has an elastic modulus of 1200 to 2800 Pa.

8. A pharmaceutical composition for preventing or treating muscle disease or muscle damage, comprising the hydrogel composition of claim 1.

9. The pharmaceutical composition according to claim 8, wherein the muscle disease is one or more muscle disease selected from muscular dystrophy, muscular atrophy, disuse skeletal muscle atrophy, sarcopenia, muscle loss, myalgia, myasthenia, muscle stiffness, ligament rupture, neurofibromatosis, tenosynovitis, polymyositis, dystonia, inflammatory muscle lesions (myositis), myotonia, metabolic muscle lesions, periodic paralysis, and congenital muscle lesions.

10. A method for preventing or treating muscle diseases, including administering or implanting the hydrogel composition of claim 1 to a subject other than a human in need of administration or implantation thereof.

11. The method for preventing or treating muscle diseases according to claim 10, wherein the muscle disease is one or more muscle disease selected from muscular dystrophy, muscular atrophy, disuse skeletal muscle atrophy, sarcopenia, muscle loss, myalgia, myasthenia, muscle stiffness, ligament rupture, neurofibromatosis, tenosynovitis, polymyositis, dystonia, inflammatory muscle lesions (myositis), myotonia, metabolic muscle lesions, periodic paralysis, and congenital muscle lesions.

12. A composition for culturing muscle organoids, comprising the hydrogel composition of claim 1.

13. A composition for implanting into muscle tissue in a subject requiring implantation of muscle cells, comprising the hydrogel composition of claim 1.
